# EUROPÄISCHE PATENTSCHRIFT

(11) **EP 0 438 540 B1**
(45) Veröffentlichungstag und Bekanntmachung des Hinweises auf die Patenterteilung: **09.11.1994**
(21) Anmeldenummer: 90904794.6
(22) Anmeldetag: 15.02.1990
(51) Int. Cl.: A61F 9/04, A47C 16/00

(54) **SCHLAFMASKE**
SLEEPING MASK
MASQUE POUR DORMIR

(30) Priorität: 17.08.1989 AT 1952/89
(43) Veröffentlichungstag der Anmeldung: 31.07.1991
(73) Patentinhaber: Adamo, Renè, A-5061 Elsbethen (AT)
(72) Erfinder: Adamo, Renè, A-5061 Elsbethen (AT)
(74) Vertreter: Schmitz, Hans-Werner, Dipl.-Ing.
(86) Internationale Anmeldenummer: EP9000247
(87) Internationale Veröffentlichungsnummer: WO9102502

(56) Entgegenhaltungen:
- DE-C- 191 792
- FR-A- 916 473
- FR-E- 44 889
- US-A- 2 148 838
- US-A- 2 243 982
- US-A- 2 342 840
- US-A- 2 667 869
- US-A- 2 671 898
- US-A- 2 696 008
- US-A- 2 942 270
- US-A- 4 790 031

## Beschreibung

Die Erfindung betrifft eine Schlafmaske nach dem Oberbegriff des Anspruches 1.

Eine derartige Schlafmaske ist aus der US-A-2 243 982 bekannt. Bei der Ausführungsform der Fig. 1 und 2 der US-A-2 243 982 wird in den Seitenteilen der Augenbinde jeweils ein bogenförmiger Schlitz angebracht, so daß sich eine C-förmige Öffnung mit einem Bügel ergibt, in die die Ohrmuschel des Benutzers eingesetzt werden kann, wodurch die bekannte Schlafmaske am Kopf des Benutzers befestigt wird. Durch den bogenförmigen Schlitz entsteht ferner ein Streifen bzw. eine Zunge, die im Tragezustand die Ohröffnung bedeckt. Die genannte Zunge kann auch zusammengeknüllt und als eine Art Stopfen in das Ohr eingesetzt werden.

Die gattungsgemäße Schlafmaske ist zunächst insofern nachteilig, als der durch den Einschnitt gebildete Bügel relativ rißgefährdet ist, da sich nur ein schmaler Steg ergibt, der insbesondere beim Überstülpen über das Ohr in hohen Maße beschädigungsgefährdet ist. Darüber hinaus ist die Abdeckung des Ohres und damit die Schalldämmung wenig zufriedenstellend. Wird die sich bildende Zunge zusammengeknüllt, kann die Schalldämmung zwar etwas verbessert werden, jedoch ergibt sich daraus wiederum der Nachteil, daß bei einer langen Benutzungsdauer der in das Ohr gesteckte Stopfen drückt und somit für den Benutzer unangenehm ist.

Aus der US-A-2 942 270 ist eine weitere Schlafmaske bekannt, bei der die Augenbinde mit Hilfe zweier kordelartiger Befestigungsschnüre an den Ohren befestigt wird. Die freien Enden der Befestigungsschnüre weisen Ohrstopfen auf, die zur Schalldämmung in das Ohr gesteckt werden. Der Hauptnachteil dieser bekannten Schlafmaske ist ein relativ geringer Tragekomfort, da die um die Ohren geführten Befestigungsschnüre einschneiden können und überdies bei dieser Schlafmaske die in die Ohren gesteckten Stopfen bei langer Benutzungszeit unangenehm sind.

Weitere aus der US-A-2 342 840, dem DE-U-8 808 220 und der DE-A-3 920 954 bekannte Schlafmasken sind mit Befestigungseinrichtungen, beispielsweise in Form eines Gummizuges versehen, die um den Hinterkopf herumgeführt werden. Hierbei ist vor allem nachteilig, daß die um den Hinterkopf geführte Befestigungseinrichtung nicht nur als störend empfunden wird, sondern auch bei einer Kopfbewegung von der Kopfauflage zurückgehalten wird, so daß es immer wieder zu einem Verrutschen der Augenbinde kommen kann. Ferner bietet die aus der US-A-2 342 840 bekannte Schlafmaske keinerlei Lärmschutz, während die beiden anderen zuletzt genannten bekannten Schlafmasken den weiteren Nachteil eines aufwendigen und somit teuer herzustellenden Aufbaues aufweisen.

Es ist daher Aufgabe der vorliegenden Erfindung, eine Schlafmaske der im Oberbegriff des Anspruches 1 angegebenen Art zu schaffen, die einfach ausgebildet ist, und einen guten, als angenehm empfundenen Sitz der Augenbinde ermöglicht, ohne daß ein Verrutschen der Augenbinde bei einer Kopfbewegung gegenüber der Kopfauflage zu befürchten wäre.

Die Lösung dieser Aufgabe erfolgt durch die Merkmale des Anspruches 1.

Durch die bis zu den Ohren verlängerten, Aufnahmetaschen für die Ohrmuscheln aufweisenden Seitenteile der Augenbinde entfällt die Notwendigkeit, die Augenbinde über eine um den Hinterkopf reichende Halterung zu schließen, weil die Augenbinde über die verlängerten Seitenteile an den Ohrmuscheln befestigt werden kann, die in die jeweiligen Aufnahmetaschen eingreifen. Der von einer Halterung für die Augenbinde freie Hinterkopf kann daher auch gegenüber einer Kopfauflage bewegt werden, ohne daß ein Verrutschen der Augenbinde befürchtet werden müßte. Die die Ohrmuscheln aufnehmenden Aufnahmetaschen werden außerdem kaum durch eine Frisur beeinträchtigt, die auch durch die Aufnahmetaschen nicht zerstört werden kann, wie dies bei sich um den Hinterkopf schließenden Gummizügen der Fall ist.

Die auf der dem Kopf zugewandten Innenseite vorgesehenen Aufnahmetaschen für die Ohrmuscheln haben in der erfindungsgemäßen Schlafmaske nicht nur einen guten Halt, sondern stellen auch eine gute Abdeckung der Ohrmuscheln dar, was eine Lärmabschirmung ergibt, die sich insbesondere gegenüber Hintergrundgeräuschen, wie Straßenlärm, Lüftergeräuschen von Klimaanlagen u.dgl. als vorteilhaft erweist. Die schalldämmende Wirkung der die Ohrmuscheln aufnehmenden Aufnahmetaschen kann erheblich gesteigert werden, wenn die verlängerten Seitenteile im Ohrbereich mit einem schalldämmenden Werkstoff versehen sind.

Zur Optimierung der Anpassung an die anatomischen Verhältnisse ist die erfindungsgemäße Schlafmaske vorzugsweise mit einem geschwungenen wellenförmig verlaufenden Außenrand versehen, so daß die Seitenteile bzw. deren Aufnahmetaschen zur vollständigen Abdeckung der Ohrmuscheln im wesentlichen rund bis oval ausgebildet sind und über taillierte, in ihrer Abmessung gegenüber den Seitenteilen verringerte Übergangsbereiche mit wiederum vergrößerten Augenabdeckbereichen verbunden sind. Die Augenabdeckbereiche wiederum sind über einen ebenfalls taillierten in seiner Abmessung verringerten Nasenbereich miteinander verbunden, der bevorzugterweise mit Auflagepolstern versehen sein kann, die sich im Tragezustand auf den Außenseiten der Nase auflegen und somit den Tragekomfort und den sicheren Halt weiter erhöhen. Ebenfalls können die Innenflächen der Aufnahmetaschen mit schalldämmenden Einlagen versehen sein, die sich im Tragezustand auf die Öffnung der Ohrmuschel legen und somit die Lärmdämmung wesentlich verbessern. Schließlich können auch die Augenbereiche mit Auflagepolstern versehen werden, die zum einen den Tragekomfort und zum anderen die Abdunklungswirkung erheblich verbessern.

Sind die Aufnahmetaschen von den jeweiligen Innenflächen der Seitenteile und einem beispielsweise aus einem flexiblen Stoff bestehenden flächigen Halteteil gebildet, ergibt sich der Vorteil, daß die Innenfläche der Augenbinde einstückig in die Seitenbereiche verlängert werden kann, was mit anderen Worten bedeutet, daß die Innenfläche der Augenbinde und der Seitenbereiche ein Teil sein kann. Auf den Ohrbereich der Seitenteile wird dann lediglich nur noch das vorzugsweise aus Stoff, wie Seide o.ä., bestehende Halteteil aufgenäht, wobei sich eine Einführöffnung ergibt, in die die Ohrmuschel zum Festlegen der erfindungsgemäßen Schlafmaske eingeführt wird. Danach umgreift die Innenfläche und das Halteteil, die die jeweilige Aufnahmetasche der Seitenteile bilden, das Ohr vollständig, so daß dieses einerseits vollständig abgedeckt ist uni andererseits im gleichen Zuge damit die Schlafmaske am Kopf des Benutzers festgelegt wird.

Zur Verbesserung des Sitzes kann hierbei der Öffnungsrand der jeweiligen Aufnahmetasche entweder versteift sein oder mit einen Zugband versehen sein, das es ermöglicht, den Öffnungsrand zusammenzuziehen und dadurch die Schlafmaske zu fixieren.

Die Unteransprüche haben vorteilhafte Weiterbildungen der Erfindung zum Inhalt.

Um die Fixierstellung zu sichern, kann das freie Ende des Zugbandes mit einem Fixierknopf versehen sein, der beispielsweise als Druckknopf ausgebildet sein kann, der ein federbelastetes Betätigungsteil aufweist, das in der Fixierstellung den jeweiligen Bereich des freien Endes des Zugbandes zusammenklemmt und damit festelegt.

Um beispielsweise mit einer Standardgröße Anpassungen an unterschiedliche Kopfabmessungen vornehmen zu können, kann der Übergangsbereich zwischen oder Augenbinde und den Seitenteilen elastisch dehnbar ausgebildet werden. Hierzu ist es möglich, den Übergangsbereich als ein elastisches Band auszubilden, das zwischen die Augenbinde und das Seitenteil eingesetzt wird. Hieraus ergibt sich der Vorteil, daß die Augenbinde unabhängig von den Kopfabmessungen ihren Dimensionierungen und der Lage der u.U. vorgesehenen Auflagepolster gleich gehalten werden kann, da eine Anpassung an unterschiedliche Kopfabmessungen über den elastischen Übergangsbereich erfolgt, der jedoch beim Ausdehnen oder Zusammenziehen die Lage der Augenbinde relativ zum Kopf unbeeinflußt läßt.

Eine Anpassung der erfindungsgemäßen Schlafmaske an die jeweiligen anatomischen Verhältnisse kann in einfacher Weise ferner dadurch sichergestellt werden, daß die Seitenteile der Augenbinde in ihrer Länge veränderbar ausgebildet sind, was mit Gummizügen auch durch Klettverbindungen, Schnallen o. dgl. erreicht werden kann.

Die Verlängerung der Augenbinde zu eine Halterung ergebenden Seitenteilen gewährleistet außerdem die Möglichkeit einer breitflächigeren Anlage der Schlafmaske im Schläfenbereich mit dem Vorteil, daß sich die Augenbinde nicht im Bereich der äußeren Augenwinkel einzieht, was den Sitz der Augenbinde erheblich verschlechtern würde. Weisen die Seitenteile eine an den durchschnittlichen Abstand zwischen dem oberen Augenbrauenwulst und der unteren Augenbegrenzung angepaßte Mindestbreite auf, so kann diese Gefahr auch bei einem übermäßigen Zug weitgehend ausgeschaltet werden, insbesondere wenn die Augenbinde im Augen- bzw. Nasenbereich mit entsprechenden Auflagepolstern versehen ist. In diesen Zusammenhang ist zu erwähnen, daß aus medizinischen Gründen eine Berührung der Hornhaut bei geöffnetem Auge möglichst verhindert werden soll. Dies kann vorzugsweise durch die erwähnten Auflagepolster erreicht werden, die sich im Tragezustand auf die Augenlider legen und weitgehend deren Öffnung verhindern können.

Weitere Einzelheiten, Merkmale und Vorteile der Erfindung ergeben sich aus nachfolgender Beschreibung von Ausführungsbeispielen anhand der Zeichnung.

Es zeigt:
- Fig. 1: eine Draufsicht auf die Innenfläche einer ersten Ausführungsform einer erfindungsgemäßen Schlafmaske,
- Fig. 2: eine Schnittdarstellung entlang der Linie II-II in Fig. 1,
- Fig. 3: eine der Fig. 1 entsprechende Darstellung einer zweiten Ausführungsform der erfindungsgemäßen Schlafmaske,
- Fig. 4: eine der Fig. 3 entsprechende vergrößerte Darstellung des rechten Teiles der Schlafmaske gemäß Fig. 3,
- Fig. 5: eine Draufsicht auf eine erfindungsgemäße Nackenstütze,
- Fig. 6: eine teilweise geschnittene Stirnansicht auf die Nackenstütze gemäß Fig. 5 aus Richtung der Pfeile VI-VI und
- Fig. 7: eine Seitenansicht der Nackenstütze gemäß den Fig. 5 und 6.

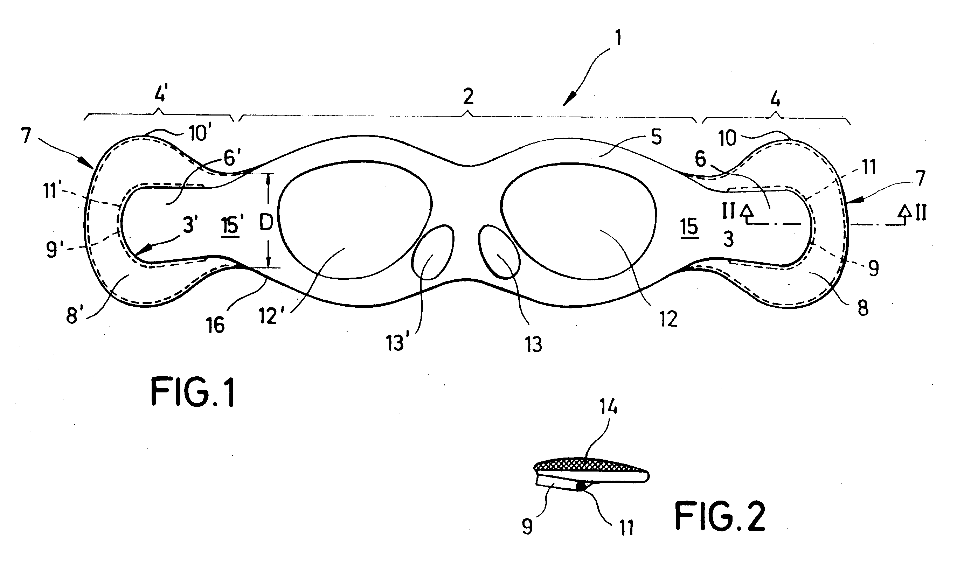

In Fig. 1 ist eine erste Ausführungsforn einer erfindungsgemäßen Schlafmaske 1 dargestellt, die eine in ihrer Gesamtheit mit 2 bezeichnete Augenbinde und eine zweiteilige Halterung 3,3' zur Befestigung der Augenbinde 2 am Kopf eines Benutzers aufweist.

Die Augenbinde 2 ist mit zwei Seitenteilen 4,4' versehen, die im Beispielsfalle einstückig mit der Augenbinde 2 verbunden sind. Die Seitenteile 4,4' schließen sich an den gegenüberliegenden Enden der Augenbinde 2 an diese an.

Die Augenbinde 2 weist eine in Fig. 1 nicht sichtbare Außenfläche und eine im Benutzungszustand der Schlafmaske 1 den Augen eines Benutzers zugewandte Innenfläche 5 auf, die sich aufgrund der einstückigen Ausbildung bei der in Fig. 1 dargestellten Ausführungsform in Innenflächen 6 bzw. 6' der Seitenteile 4 bzw. 4' fortsetzt. Entsprechend setzt sich die in Fig. 1 nicht sichtbare Außenfläche der Augenbinde 2 in die ebenfalls nicht sichtbaren Außenflächen der Seitenteile 4,4' fort.

Die Seitenteile 4,4' weisen ferner jeweils eine Befestigungseinrichtung zum Festlegen der Augenbinde 2 an den Ohrmuscheln eines Benutzers auf, die als Aufnahmetaschen 7 bzw. 7' ausgebildet sind. Wie Fig. 1 verdeutlicht, sind die Aufnahmetaschen 7,7' jeweils von der Innenfläche 6 bzw. 6' der Seitenteile 4,4' und einen Halteteil 8 bzw. 8' gebildet. Fig. 1 verdeutlicht ferner, daß die Halteteile 8,8' im Beispielsfalle als ein im wesentlichen C-förmiger Streifen, vorzugsweise aus Stoff, wie Seide o.ä., ausgebildet sind. Die Halteteile 8,8' weisen hierbei einen freien nicht befestigten Öffnungsrand 9 bzw. 9' und einen mit dem Seitenteil 4 bzw. 4' fest verbundenen, vorzugsweise vernähten, Außenrand 10,10' auf. Die in Fig. 1 gestrichelte Linie im Bereich der Außenränder 10 bzw. 10' verdeutlicht hierbei die um den gesamten Außenrand 10 herum verlaufende Naht, die das tuchförmige Halteteil 8 bzw. 8' auf der Innenfläche 6,6' der Seitenteile 4,4' festlegt. Hieraus entstehen die beutelartigen Aufnahmetaschen 7,7', die derart dimensioniert sind, daß sie die Ohrmuschel eines Benutzers im wesentlichen vollständig umschließen können.

Bei der in Fig. 1 dargestellten Ausführungsform ist der Öffnungsrand 9 bzw. 9' versteift, wozu er beispielsweise eine eingenähte Versteifung 11 bzw. 11', beispielsweise aus Fischbein, aufweisen kann.

Zur besseren Anpassung der Augenbinde 2 bzw. Schlafmaske 1 an die anatomischen Verhältnisse kann die Augenbinde 2 im Augen- und Nasenbereich mit Auflagepolstern 12 bzw. 12' für die Augen und 13,13' für die Auflage auf den Nasenseitenflächen versehen werden.

Um eine gute Schalldämmung zu erreichen, können darüber hinaus die Seitenteile 4,4' im Ohrbereich, also im Bereich der Aufnahmetaschen 7,7' mit einer Einlage aus schalldämmendem Werkstoff 14 versehen sein, wie sich dies aus der Schnittdarstellung der Fig. 2 ergibt.

Die Längenanpassung der Schlafmaske 1 an den entlang der Augenbinde gemessenen Ohrenabstand kann durch eine entsprechende Längenverstellung der Seitenteile 4,4' erfolgen, die zu diesen Zweck mit einem gegebenenfalls unter einer federnden Zugspannung stehenden Faltenzug ausgerüstet werden können.

Ferner ist es möglich, den Übergangsbereich 15 bzw. 15' zwischen der Augenbinde 2 und den Seitenteilen 4,4' elastisch dehnbar auszubilden. Hierzu kann der Übergangsbereich 15,15' als ein elastisches Band ausgebildet sein, das zwischen die Endbereiche der Augenbinde 2 und die anschließenden Teile der Seitenteile 4 bzw. 4' eingenäht wird.

Die Breite der Seitenteile 4,4' sollte im Ohrbereich an die Ohrgröße angepaßt sein, nicht aber im Übergangsbereich 15, 15' zur Augenbinde 2 hin. Um ein Einziehen der Augenbinde 2 im Bereich der äußeren Augenwinkel sicher zu vermeiden, empfiehlt es sich, die Mindestbreite D der Seitenteile 4,4' an den durchschnittlichen Abstand zwischen dem oberen Augenbrauenwulst und der unteren Augenbegrenzung anzupassen, so daß sich eine ausreichend große, flächige Anlage in Schläfenbereich ergibt.

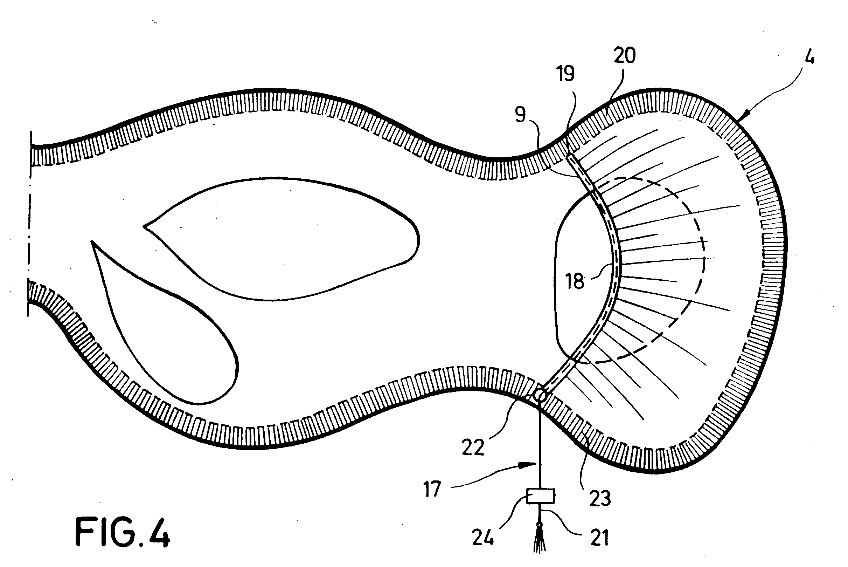

Fig. 1 verdeutlicht ferner, daß der um die gesamte Schlafmaske 1 umlaufende Außenrand 16 wellenförmig verläuft, wobei sich Vergrößerungen der Abmessungen im Bereich der Seitenteile 4,4' und der Innenflächenbereiche der Augenbinde 2 ergeben, die die Auflagepolster 12,12' für die Augen aufweisen. Der wellenförmige Verlauf des Randes 16 ergibt sich hierbei aus den in Fig. 4 angegebenen Maßen B,C,D und E, die für Herren und Damen in der nachstehenden Tabelle wiedergegeben sind:

| | Herrengröße | Damengröße |
|---|---|---|
| A | 20,5 | 19,0 |
| B | 3,5 | 3,5 |
| C | 8,5 | 8,5 |
| D | 5,5 | 5,5 |
| E | 9,0 | 9,0 |
| F | 14,0 | 13,5 |
| G | 13,5 | 13,0 |

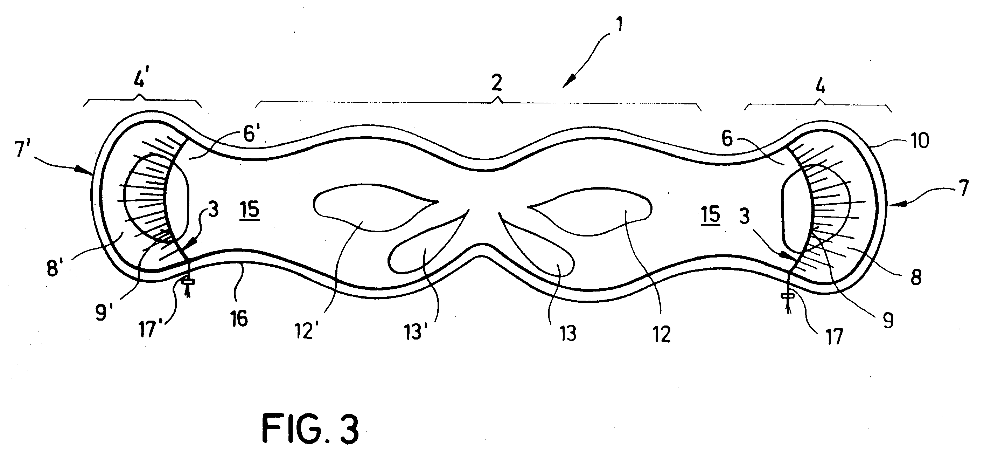

Die in dieser Tabelle aufgeführten Maße sind alle in cm angegeben und können sich sowohl auf die Ausführungsform gemäß Fig. 4 bzw. Fig. 3 wie auch auf die Ausführungsform der Fig. 1 und 2 beziehen. Ferner stellen diese Maße lediglich ein Beispiel gängiger Größen für Mitteleuropäer dar. Jegliche Art von Anpassungen sind aber möglich.

Die Fig. 1, 3 und 4 verdeutlichen hierbei ferner, daß die in ihren Abmessungen jeweils verringerten Bereiche zwischen den Seitenteilen 4,4' und der Augenbinde 2 und zwischen den Augenauflagebereichen 12,12' und dem Nasenauflagebereich 13, 13' ausgerundet sind, so daß sich die bereits angesprochene Wellenform ergibt. Diese Wellenform hat besonders hohen Tragekomfort und ist an die anatomischen Verhältnisse sehr gut angepaßt.

Die Ausführungsform gemäß den Fig. 3 und 4 entspricht im wesentlichen derjenigen der Fig. 1 und 2, so daß zur Bezeichnung gleicher Teile dieselben Bezugszeichen wie in den Fig. 1 und 2 verwendet wurden.

Zu den Hauptunterschieden zwischen den beiden Ausführungsformen zählt, daß zum einen das Halteteil 8 bzw. 8' vollflächiger ausgestaltet ist und mithin eher die Form eines Streifens mit abgerundeten Längsrändern aufweist, wie dies im einzelnen aus der Darstellung der Fig. 3 hervorgeht.

Ferner ist der Öffnungsrand 9 bzw. 9' der Aufnahmetaschen 7 bzw. 7' mit einem Zugband 17 bzw. 17' versehen. Dieses wird nachfolgend unter Bezugnahme auf Fig. 4 beschrieben, wobei nur auf das Zugband 17 Bezug genommen wird, da dieses in gleicher Weise wie das Zugband 17' ausgebildet ist. Aus der Darstellung der Fig. 4 ergibt sich, daß das Zugband 17 entlang des Öffnungsrandes 9 verläuft und hierbei in einem Saum 18 angeordnet ist, der entlang des Öffnungsrandes 9 verläuft und einen Aufnahmekanal für das Zugband 17 bildet. Ein Ende 19 des Zugbandes 17 ist hierbei an oberen Rand 20 des Seitenteiles 4 befestigt, während das freie Ende 21 aus einer Ausnehmung 22 im unteren Rand 23 des Seitenteiles 4 herausgeführt ist. Das freie Ende 21 ist mit einem Fixierknopf 24 versehen, der die jeweilige Fixierstellung des Zugbandes 17 festlegen kann.

Die in den Fig. 1 - 4 dargestellten Ausführungsformen der erfindungsgemäßen Schlafmaske 1 können in unterschiedlichen Qualitäten hergestellt werden. So ist es möglich, eine mehr oder weniger als Einwegversion verwendbare Ausführung zu schaffen, bei der weitgehend billige Werkstoffe verwendet werden, so daß die Schlafmaske 1 nach einmaligen Gebrauch weggeworfen werden kann.

Ferner ist es jedoch auch möglich, die Schlafmaske 1 in einer vollständig waschbaren Ausführungsform herzustellen, die mehrfach gebraucht werden kann. Bevorzugte Werkstoffe stellen hierbei insbesondere Leder für die Augenbinde und die Seitenteile und Stoff, wie insbesondere Seide, für die Halteteile dar.

Soll die Schlafmaske 1 benutzt werden, wird diese mit der Augenbinde 2 auf den Augen und Nasenbereich aufgesetzt und danach an den Ohrmuscheln festgelegt, wozu diese durch die vom Öffnungsrand 9,9' begrenzte Einführöffnung in die Aufnahmetaschen 7,7' eingeführt werden. Hierbei hält entweder der versteifte Öffnungsrand oder das festgezogene Zugband 17,17' die Ohrmuscheln fest in den Aufnahmetaschen 7,7'.

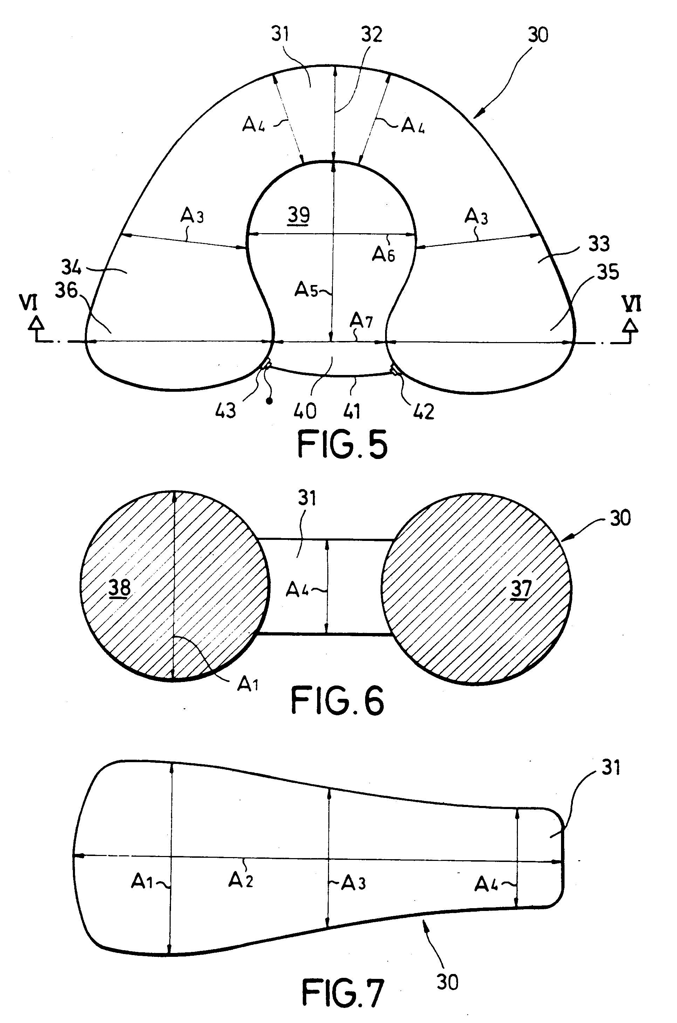

In den Fig. 5 - 7 ist eine vorzugsweise aufblasbare erfindungsgemäße Nackenstütze 30 dargestellt, die eine Taille 31 sowie zwei sich spiegelsymmetrisch zur Taillenquerschnittsfläche 32 in die gleiche Richtung erstreckende Seitenschenkel 33 und 34 aufweist. Fig. 5 zeigt hierbei, daß sich durch diese Ausbildung eine im wesentlichen U- bzw. V-förmige Konfiguration ergibt. Die Seitenschenkel 33 und 34 weisen stirnseitige Endbereiche 35 bzw. 36 auf, deren in Fig. 6 dargestellte Querschnittsfläche 37 bzw. 38 vorzugsweise kreisförmig ist. Ferner ist die Abmessung der Endbereiche 35 und 36 bzw. deren Querschnittsfläche 37 und 38 zumindestens 1,5-mal so groß wie die Querschnittsabmessung der Taille 31. Bei der in den Fig. 5 - 7 dargestellten Ausführungsform beträgt die Querschnittsabmessung vorzugsweise das 2-fache der Querschnittsabmessung der Taille 31. Hieraus ergibt sich eine vor allem aus Fig. 7 ersichtliche allmähliche Verdickung der Seitenschenkel 33,34 von der Taille 31 aus. Hierdurch wird ein im wesentlichen kreisförmiger Halsaufnahmebereich 39 mit einem in seiner Abmessung verengten Einführbereich 40 geschaffen, was insbesondere auch aus der Draufsicht aus Fig. 5 ersichtlich ist.

Fig. 5 zeigt ferner, daß die Endbereiche 35 und 36 über eine vorzugsweise längenverstellbare Schnur 41 miteinander verbunden sind, die über Befestigungsteile 42 und 43 gehalten ist, wobei das Befestigungsteil 43 eine vorzugsweise selbsthemmende Durchführöffnung hat, die die Schnur 41 im jeweiligen Einstellzustand fixiert.

Eine besonders bevorzugte jedoch nur ein mögliches Beispiel darstellende Ausführungsform der in den Fig. 5 bis 7 dargestellten Nackenstütze 30 weist die in diesen Figuren angegebenen Bemessungen A1 -A7 auf, wobei sich die Bemessungen aus nachstehender Tabelle ergeben.

| | |
|---|---|
| A1 | = 14 |
| A2 | = 23,5 |
| A3 | = 10 |
| A4 | = 7 |
| A5 | = 13 |
| A6 | = 13 |
| A7 | = 8. |

Alle Bemessungen sind hierbei in cm angegeben.

Die erfindungsgemäße Nackenstütze 30 ergibt den Vorteil, daß insbesondere beim Sitzschlaf der Kopf des Benutzers, dessen Hals im Aufnahmebereich 39 angeordnet ist, fixiert ist, was einen hohen Tragekomfort und einen guten Schlaf ergibt.

Ferner können die in den Fig. 1 - 4 beschriebene Schlafmaske 1 und die Nackenstütze 30 vorzugsweise in Kombination benutzt werden, woraus sich ein Schlafhilfesystem ergibt, das insbesondere für Langstreckenreisende auf Langstreckenflügen, in Automobilen, auf Schiffen oder in Zügen als besonders vorteilhaft herausgestellt hat. Jedoch auch die Benutzung nur der Schlafmaske 1 oder der Nackenstütze 30 ergibt für die genannten Reisearten bereits erhebliche Vorteile gegenüber herkömmlichen Schlafmasken und Nackenstützen.

## Patentansprüche

1. Schlafmaske (1)
- mit einer Augenbinde (2), die eine Außenfläche und eine im Benutzungszustand den Augen zugewandte Innenfläche (5) aufweist; und
- mit einer Halterung (3,3') zur Befestigung der Augenbinde (2) am Kopf eines Benutzers, die zwei Seitenteile (4,4') aufweist, die sich an die gegenüberliegenden Enden der Augenbinde (2) anschließen und Aussen- und Innenflächen (6,6') in Verlängerung der Aussen- und Innenflächen (5) der Augenbinde (2) aufweisen und die jeweils eine Befestigungseinrichtung zum Festlegen der Augenbinde (2) an den Ohrmuscheln des Benutzers aufweisen; dadurch gekennzeichnet,
- daß die Befestigungseinrichtungen als Aufnahmetaschen (7,7') ausgebildet sind, die jeweils von der Innenfläche (6,6') der Seitenteile (4,4') und einem Halteteil (8,8') gebildet werden, das einen freien Öffnungsrand (9,9') und einen mit dem Seitenteil (4,4') fest verbundenen Außenrand (10,10') aufweist, und
- daß der Öffnungsrand (9,9') mit einem Zugband (17,17') versehen ist, das durch einen entlang des Öffnungsrandes (9,9') verlaufenden Saum (18) geführt ist und dessen eines Ende (19) am oberen Rand (20) des Seitenteiles (4) befestigt ist, während sein freies Ende (21) durch eine Ausnehmung (22) im unteren Rand (23) des Seitenteiles (4) herausgeführt ist.

2. Schlafmaske nach Anspruch 1, dadurch gekennzeichnet, daß der Öffnungsrand (9,9') versteift ist.

3. Schlafmaske nach Anspruch 1, dadurch gekennzeichnet, daß das freie Ende (21) mit einem Fixierknopf (24) versehen ist.

4. Schlafmaske nach einem der Ansprüche 1 - 3, dadurch gekennzeichnet, daß die Seitenteile (4,4') in ihrer Länge veränderbar sind.

5. Schlafmaske nach Anspruch 4, dadurch gekennzeichnet, daß ein Übergangsbereich (15,15') zwischen der Augenbinde (2) und den Seitenteilen (4,4') elastisch dehnbar ist.

6. Schlafmaske nach einem der Ansprüche 1 - 5, dadurch gekennzeichnet, daß die Übergangsbereiche (15,15') eine an den durchschnittlichen Abstand zwischen dem oberen Augenbrauenwulst und der unteren Augenbegrenzung angepaßte Mindestbreite (D) aufweisen.

7. Schlafmaske nach Anspruch 1 oder 2, dadurch gekennzeichnet, daß die Augenbinde (2) zwei Augenbereiche aufweist, die mit Auflagepolstern (12) versehen sind, und daß die Augenbereiche über einen Nasenbereich miteinander verbunden sind, der ebenfalls mit Auflagepolstern (13,13') versehen ist.

## Claims

1. A sleeping mask (1), comprising
- an eye mask (2) including an outer surface and an inner surface (5) facing the eyes in the state of use, and
- a holding device (3,3') for securing said eye mask (2) to the head of a user, said eye mask (2) including two side parts (4, 4') which join the opposite ends of said eye mask (2) and comprise outer and inner surfaces (6, 6') in extension of said outer and inner surfaces (5) of said eye mask (2) and of which each comprises a fastening means for securing said eye mask (2) to the outer ears of the user, characterized in
- that said fastening means are formed as receiving pockets (7, 7'), each of said receiving pockets (7, 7') being formed by said inner surface (6, 6') of said side parts (4, 4') and by a holding member (8, 8') having a free opening edge (9, 9') and an outer edge (10, 10') connected firmly to said side part (4, 4') and
- that said opening edge (9, 9') is provided with a drawstring (17, 17') which is passed through a seam (18) extending along said opening edge (9, 9') and whose one end (19) is secured to the upper edge (20) of said side part (4), whilst its free end (21) projects out a recess (22) provided in the lower edge (23) of said side part (4).

2. A sleeping mask as defined in claim 1, characterized in that said opening edge (9, 9') is stiffened.

3. A sleeping mask as defined in claim 1, characterized in that said free end (21) is provided with a fixing button (24).

4. A sleeping mask as defined in any of claims 1 - 3, characterized in that the length of said side parts (4, 4') is variable.

5. A sleeping mask as defined in claim 4, characterized in that a transition portion (15, 15') provided between said eye mask (2) and said side parts (4, 4') is elastically stretchable.

6. A sleeping mask as defined in any of claims 1 - 5, characterized in that said transition portions (15, 15') have a minimum width (D) adapted to the average distance between the upper line of the eyebrow and the lower edge of the eye.

7. A sleeping mask as defined in claim 1 or 2, characterized in that said eye mask (2) comprises two eye portions which are provided with contact pads (12), and that said eye portions are interconnected through a nose portion which is also provided with contact pads (13, 13').

## Revendications

1. Masque pour dormir (1)
- avec un bandeau pour les yeux (2), qui présente une surface extérieure et une surface intérieure (5) orientée, en position d'utilisation, vers les yeux, et
- avec un support (3,3') pour la fixation du bandeau pour les yeux (2) sur la tête d'un utilisateur, qui présente deux éléments latéraux (4, 4') qui sont adjacents aux extrémités opposées du bandeau pour les yeux (2) et présentent des surfaces extérieure et intérieure (6,6') en prolongement des surfaces extérieure et intérieure (5) du bandeau pour les yeux (2) et qui présentent, chacun, un dispositif de fixation pour la fixation du bandeau pour les yeux (2) sur les pavillons d'oreille de l'utilisateur; caractérisé en ce
- que les dispositifs de fixation se présentent sous forme de poches de réception (7,7') qui sont formées, chacune, par la surface intérieure (6, 6') des éléments latéraux (4,4') et un élément de retenue (8,8') qui présente un bord d'ouverture libre (9,9') et un bord extérieur (10,10') solidaire de l'élément latéral (4,4'), et
- que le bord d'ouverture (9,9') est pourvu d'un ruban de traction (17,17') qui est conduit à travers un ourlet (18) s'étendant le long de bord d'ouverture (9,9') et dont l'une des extrémités (19) est fixée au bord supérieur (20) de l'élément latéral (4), tandis que son extrémité libre (21) ressort à travers un évidement (22) dans le bord inférieur (23) de l'élément latéral (4).

2. Masque pour dormir suivant la revendication 1, caractérisé en ce que le bord d'ouverture (9,9') est raidi.

3. Masque pour dormir suivant la revendication 1, caractérisé en ce que l'extrémité libre (21) est pourvue d'un bouton de fixation (24).

4. Masque pour dormir suivant l'une des revendications 1 à 3, caractérisé en ce que les éléments latéraux (4,4') sont modifiables en longeur.

5. Masque pour dormir suivant la revendication 4, caractérisé en ce qu'une zone de transition (15,15') entre le bandeau pour les yeux (2) et les éléments latéraux (4,4') est élastiquement extensible.

6. Masque pour dormir suivant l'une des revendications 1 à 5, caractérisé en ce que les zones de transition (15,15') présentent une largeur minimale (D) adaptée à la distance moyenne entre le rebord supérieur des sourcils et la délimitation inférieure des yeux.

7. Masque pour dormir suivant la revendication 1 ou 2, caractérisé en ce que le bandeau pour les yeux (2) présente deux zones oculaires qui sont pourvues de tampons d'appui (12) et que les zones oculaires sont reliées l'une à l'autre par une zone de nez qui est également pourvue de tampons d'appui (13, 13').
